# EUROPEAN PATENT APPLICATION

(11) **EP 4 169 502 A1**
(43) Date of publication of application: **26.04.2023**
(21) Application number: 21382951.8
(22) Date of filing: 22.10.2021
(51) Int. Cl.: A61K 8/9789, A61L 27/50, A61K 36/00, A61P 17/00, A61Q 19/00

(54) **COSMETIC AND DERMATOLOGICAL USE OF RUBUS FRUTICOSUS FRUIT EXTRACT**

(71) Applicant: Provital, S.A.U., 08010 Barberà del Vallès (ES)
(72) Inventor: RUBIO CAÑADAS, Emilio, 08380 MALGRAT DE MAR (ES); ASO PÉREZ, Miguel, 08191 RUBÍ (ES); MANZANO ALÍAS, David, 08011 BARCELONA (ES)
(74) Representative: Sugrañes, S.L.P.

(57) **Abstract**

The present invention relates to a cosmetic and dermatological use of a *Rubus fruticosus* fruit extract (RFE) for increasing adipocytic mass in adipose tissue. It relates also to a cosmetic method therefor comprising the step of topically applying to skin the RFE or a cosmetic composition comprising the RFE.

## Description

### Technical Field

The present invention relates to the cosmetic and dermatological use of a fruit extract for enhancing the mass adipose tissue, causing a volumizing effect.

### Background art

Adipose tissue is commonly known as body fat. It is found all over the body: under the skin (subcutaneous fat), packed around internal organs (visceral fat), between muscles, within bone marrow and in breast tissue.

Adipose tissue is known to be a very important and active endocrine organ. It is well established that adipocytes, fat cells, play a vital role in the storage and release of energy throughout the human body. The adipose tissue also plays an endocrine function by producing certain hormones, which regulate glucose, cholesterol and the metabolism of sex hormones.

Precursor cells of adipocytes (pre-adipocytes) localized in the adipose tissue can, under the effect of different stimuli, multiply and differentiate themselves into adipocytes. Adipocytes, differentiated cells without any capacity of division, can increase their faculty of storage by increasing the quantity of fats stored as triglycerides (lipogenesis mechanism) or conversely mobilize its stored fats by a lipolysis mechanism leading to the liberation of fatty acids and glycerol and/or mono-, diester- glycerol. Usually, there is a balance between lipogenesis and lipolysis in order to ensure stability of fatty mass.

The adipose tissue constitutes a layer of fat reserve which completely envelops the body, but whose thickness is variable. By its importance and its heterogeneous distribution, this tissue participates to the general appearance of the figure and the face. It is localised in particular in breasts, buttocks, cheeks, hands, face, neckline, eyelids, and lips. Different events, such as illness, a medical treatment, a too severe diet or old age, can involve a decreasing of this adipose tissue or lipoatrophy, which manifests itself with a thin, empty, lined and tired face.

In today's society, physical care has increased exponentially in recent years. The new trend of the so-called "body cult" affects all sectors, social classes and ages, although unevenly depending on gender and age. Everyone aspires to preserve a well-shaped figure, without localized excesses, while maintaining subcutaneous volumes fully filed.

With age, many changes occur in the skin, which lead to an aged appearance. The loss of facial volume and subcutaneous fat has been found to have a greater influence in skin aging than previously thought. As the skin of a person ages, there is a loss of facial subcutaneous fat, as disclosed in E. D. Buckingham, Poly-L-lactic acid facial rejuvenation: an alternative to autologous fat?, Facial Plast. Surg. Clin. North Am., 2013, 21(2), 271-284 and in V. Ilankovan, Upper face rejuvenation, Int. J. Oral Maxillofac. Surg., 2013, 42(4), 423-31. This loss means that, as people age, facial folds and wrinkles become more accentuated. Throughout this evolution, the process of adipogenesis (differentiation of the cells to mature adipocytes) is of fundamental importance. During youth, every stage of adipogenesis is able to proceed without complications to give a homogeneous subcutaneous adipose tissue. But as people age, preadipocytes are not able to become functional adipocytes, capable of synthesizing and accumulating fat, as disclosed in Niemela et al., Topics Tissue Eng., Eds. N-Ashammakhi, R. Reis, 2008, 4, Chapter 4, and, consequently, subcutaneous fat is getting lost. In fact, it has been seen that preadipocytes obtained from the elderly accumulate less fat and show less lipogenic enzyme activity compared to those preadipocytes from young people.

In particular, for women, breast care takes on great importance. It is one of the maximum references of femininity and one of the main symbols of feminine beauty. Although it is true that there is an important genetic component among the factors that determine the volume of the bust, there are other aspects that can influence (diet, exercise, loss of fat tissue, pregnancy, lactation, ...). For this reason, the concern for the bust is associated to women of all ages. Various studies show that women who are dissatisfied with their breast size have less vitality and optimism. The loss of self-esteem and confidence, a low mood and difficulties to relate are an example of its consequences. Thus, both personal and professional life can be affected by this discontent.

Different proposals have been put in practice to increase the volume of specific parts of the human body. Surgery, through breast implants, is the most drastic solution. However, this technique has significant disadvantages: high cost, aggressiveness (intrusion into the tissue) and risk (it is a surgical intervention). Likewise, the incorporation of foreign material to the body can cause inconvenience *a posteriori,* create interference in diagnostic methods (mammograms) and create problems in future lactations.

A recent alternative to surgery is filling by injecting substances, such as hyaluronic acid, fat or collagen. It is a less aggressive method than surgery, but it also involves an invasive action on the structure and natural composition of specific parts of the body. In addition, the increase is only temporary, and the insertion must be repeated periodically to keep the same volume, due to normal enzymatic degradation.

Another approach is the use of cosmetic substances that could provide similar effects by increasing the quantity of adipocytes and/or the accumulation of lipids in adipocytes. Different products have been disclosed in prior art addressed to obtain said effects.

For example, the activity of a commercial product is based on palmitoyl isoleucine, a synthetic lipoamino acid, which stimulates adipocyte differentiation and the increase of their volume, and which has the ability to fill wrinkles and reshape volume of cheeks.

Peptides as active ingredients have also been disclosed for enhancing adipogenesis in adipose tissue.

For example, a commercial product comprises an aqueous solution of a synthetic peptide (acetyl hexapeptide 38, Cas Nr. 1400634-44-7), which stimulates the expression of PGC-1a (peroxisome proliferator-activated receptor-gamma coactivator), enhancing the adipogenesis leading to an accumulation of lipids in adipose tissue.

In CN-A-104398399 it is disclosed a glycopeptide cream for breast enhancement and beautification, which comprises specific amounts of sheep placenta protein peptide, *Oviductus ranae* small molecular peptide, royal jelly protein peptide, collagen peptide, and a biological polysaccharide. Also, in CN-A-104606668 it is disclosed a breast enlargement gel containing complex polypeptides.

Plant extracts and essential oils have been also proposed for increasing adipocytes mass in specific parts of the body.

A commercial product contains macelignan as cosmetic ingredient. It is a lignan found in the nutmeg (*Myristica fragrans*)*,* and it is marketed for breast enhancement.

The active ingredient of a commercial product is sarsasapogenin, a phytosterol extracted from *Anemarrhena asphodeloides.* According to WO-A-2008/015639, sarsasapogenin is able to produce cosmetic lipo-filling by means of a cutaneous filling provided by an increase of the adipocytic mass neoproduced by the organism itself.

The active component of a commercial product comprises a combination of *Pyrus cydonia* seed extract, *Garcinia mangostana* peel extract, and *Chlorella vulgaris*/*Lupinus albus* protein ferment, and supports the natural process of lipid deposition lending fullness of the bust.

The *Arnica montana* flower extract is able to activate adipocytes and it is disclosed as a novel volume up cosmetic ingredient in Sakamoto et al., Discovery of a novel volume-up cosmetic ingredient: Arnica montana L. flower extract activating adipocytes, IFSCC Congress, Yokohama, 2020, Poster 18.

A commercial product comprises a *Thymus vulgaris* flower/leaf extract, which provides a skin lipo-filling stimulation, improving wrinkles and facial contour through the induction of adipogenesis.

The active component of a commercial product is a natural hydroglycolic extract from fruits of the sacred African tree *Kigelia africana.* The hormone-like substances comprised in the extract create a depth-effect, which tones and firms the bust, lift the chest, and improve skin firmness and elasticity.

A commercial product comprises an extract of *Lycium barbarum* fruit (goji berries) obtained from subcritical water extraction, which stimulates neosynthesis of lipids and maintains the skin barrier properties.

In CN-A-102670446 it is disclosed a breast enlarging and beautifying additive for cosmetics, which comprises a combination of specific amounts of *Kigelia africana* fruit extract, black soybean estrogen, *Commiphora mukul* resin, hops extract, *Leonurus sibiricus* extract, sage essential oil, *Cananga odorata* essential oil and basil essential oil.

In CN-A-107582461 it is disclosed a cosmetic composition with a breast enlarging effect, which consists of specific amounts of lupulus extract, *Hypericum perforatum* extract, *Salvia officinalis* extract and *Saponaria* leaf/root extract.

In CN-A-110051569 it is disclosed a breast enhancement composition comprising specific amounts of grape seed oil, olive fruit oil, almond oil, geranium oil, rosehip oil, *Camellia* seed oil parts, fennel oil, and jasmine flower oil.

Despite the various proposals available in the state of the art, there is still a need to have new methods for enhancing adipose tissue mass in specific parts of the human body.

### Object of the invention

The object of the present invention is the cosmetic and dermatological use of a *Rubus fruticosus* fruit extract (RFE).

Another aspect of the invention is a cosmetic and dermatological method.

### Figures

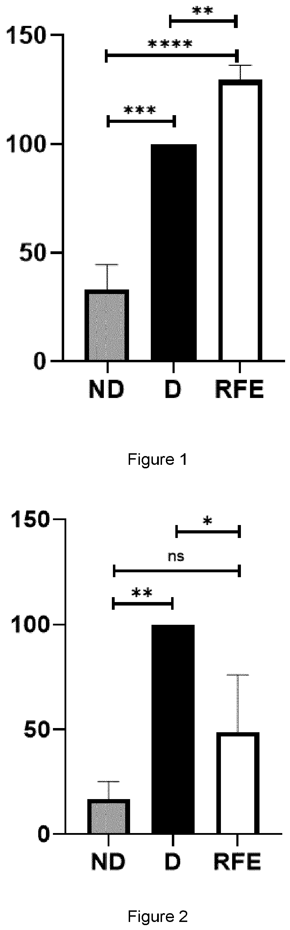

Figure 1 shows the results of the treatment of adipocytes with the RFE of the invention, according to Example 3 (Adipogenesis and lipogenesis assessment). In ordinates, the percentage of variation of the absorbance at 500 nm relative to Differentiated Control (DC) is represented. In abscises, the three groups: non-differentiated cells (ND), cells stimulated with differentiation media (DM), and cells treated with differentiation media and RFE are represented. It can be seen that the lipid content was significantly increased by 29% in adipocytes treated with the RFE used according to the invention. Statistical analysis performed by One Way ANOVA, followed by Tukey's post- test. Significant results vs Control are marked with *(p<0.05), **(p<0.01), ***(p<0.001), ****(p<0.0001).

Figure 2 shows the results of the treatment of adipocytes with the RFE of the invention, according to Example 4 (Lipolysis assessment). In ordinates, the percentage of variation of the absorbance at 570 nm relative to Differentiated Control (DC) is represented. In abscises, the three groups: non-differentiated cells (ND), cells stimulated with differentiation media (DM), and cells treated with differentiation media and RFE are represented. It can be seen that the cells stimulated with differentiation media show a 5-fold increase of glycerol release, when compared to non-differentiated cells, thus confirming the normal metabolic activity of mature adipocytes. However, differentiated cells treated with RFE significantly decrease (51%) the release of glycerol. Statistical analysis performed by One Way ANOVA, followed by Tukey's post- test. Significant results vs Control are marked with *(p<0.05), **(p<0.01), ***(p<0.001), ****(p<0.0001).

### Detailed description of the invention

The object of the present invention is the cosmetic and dermatological use of a *Rubus fruticosus* fruit extract for increasing adipocytic mass by topical application.

The authors of the present invention have surprisingly found that the topical administration of a *Rubus fruticosus* fruit extract (RFE) can effectively induce an increase of adipogenesis and/or lipogenesis, and a reduction of lipolysis in adipose tissue, leading to a significant and noticeable increasing of the adipocytic mass, which has a significant volumizing effect in specific parts of the human body, such as, for example, breasts, buttocks, cheeks, hands, face, neckline, eyelids, and lips. The topical administration of RFE surprisingly provides a cosmetic lipo-filling effect without any invasive treatment, such as surgery or injections.

In the present description, as well as in the claims, the singular forms "a", "an" and "the" include the plural reference unless the context clearly indicates otherwise. The ranges defined by the terms "between ... and ..." or by the terms "from ...to..." include also the two ends thereof. The term "about" refers to a deviation of plus/minus 10 %, preferably plus/minus 5 %. The percentages are expressed in % by weight (wt%), unless stated the contrary.

### Use of RFE

The object of the present invention is the cosmetic and dermatological use of a *Rubus fruticosus* fruit extract for increasing adipocytic mass in adipose tissue by topical application.

As used herein, "cosmetic and dermatological use" is defined as a product intended to be applied to the human skin for cleansing, beautifying, promoting attractiveness, or altering the appearance without affecting the body's structure or functions. In an embodiment, it is defined as a product intended to improve and/or embellish any part of the body showing a deficit in lipids, such as, for example, wrinkles on face or hands, breasts, neckline, buttocks, lips, cheeks, or eyelids.

As used herein, "topical application" means directly laying on or spreading on outer skin using, e.g., the hands or an applicator such as a wipe, puff, roller, spray, or cosmetotextiles. Cosmetotextiles are textiles, which provide cosmetic and biological functions, such as pleasant feeling, energising, slimming, refreshing, vitalising, skin glowing, anti-ageing, body care, fitness and health.

The increase of adipocytic mass is the result of different processes such an increase of adipogenesis and/or lipogenesis, and the reduction of lipolysis.

In the context of the present invention, adipogenesis means the process during which fibroblast like preadipocytes developed into mature adipocytes and accumulate as adipose tissue at various sites in the body.

In the context of the present invention, lipogenesis is the metabolic process through which triglycerides are formed for storage in fat.

In the context of the present invention, lipolysis is the process by which fats are broken in adipose tissue releasing fatty acids and glycerol.

In an embodiment of said use, the RFE is for increasing adipocytic mass by stimulating adipogenesis and/or lipogenesis, and reducing lipolysis in adipose tissue.

In an embodiment of said use, the RFE is for increasing adipocytic mass by stimulating adipogenesis and reducing lipolysis in adipose tissue.

In an embodiment of said use, the RFE is for increasing adipocytic mass by stimulating lipogenesis and reducing lipolysis in adipose tissue.

In a preferred embodiment of the use, the increase of adipocytic mass has a volume increasing effect of the adipose tissue.

In a preferred embodiment of the use, the topical application is to the breasts, and/or the low neckline, and/or the buttocks, and/or the face, and/or the lips, and/or the cheeks, and/or the eyelids, and/or the hands of a person in need.

The RFE for the use according to the invention increases adipogenesis and/or lipogenesis, and reduces the lipolysis, as shown by the results of *in vitro* experiments disclosed in Examples 3 and 4, in comparison to the control experiment.

The assessment of the performance of the *Rubus fruticosus* fruit extract of the invention can be done by evaluating the adipogenic effect of RFE using, for example, a test on 3T3-L1 preadipocytes. Said preadipocytes is a cell model widely used to study adipogenesis and lipogenesis, as disclosed, for example in Kassotis et al., Characterization of Adipogenic Chemicals in Three Different Cell Culture Systems: Implications for Reproducibility Based on Cell Source and Handling, Sci. Rep., 2017, 7, 42104. Adipogenesis is cell differentiation process by which pre-adipocytes become mature adipocytes (lipocytes, fat cells). Along this process the cells develop, among other metabolic changes, the capacity to produce and storage triglycerides in lipid vacuoles. The assay reproduces the differentiation process that takes place naturally in the adipose tissue to generate new adipocytes and to increase the quantity of subcutaneous fat. The assessment of adipogenesis and/or lipogenesis is based in the amount of triglycerides accumulated in the cells, which is considered a good biochemical marker for differentiation endpoint.

The use of the RFE of the invention induces a significant accumulation of lipids on adipocytes treated with said extract compared to adipocytes, which are not treated with said RFE.

By means of confocal microscopy it can be illustrated and visualized the adipogenic and lipogenic effect achieved by RFE, wherein lipids accumulate significantly in the adipocytes treated using RFE.

Another test to assess the performance of the *Rubus fruticosus* fruit extract can be done by evaluating the effect of RFE on lipolysis on 3T3-L1 preadipocytes that had been differentiated into adipocytes.

The assessment of lipolysis is based in the amount of glycerol released by the differentiated cells. Lipolysis is a catabolic process where triglycerides are hydrolysed into glycerol and free fatty acids by adipocyte lipases. Free fatty acids are then released from the adipose tissue and can be used by other body cells to produce energy.

The use of RFE significantly reduces the glycerol release from adipocytes, compared to adipocytes, which are not treated with said RFE.

Surprisingly, the RFE shows significant activity by increasing adipogenesis and/or lipogenesis and reducing lipolysis of adipocytes.

Preliminary results of *in vivo* tests confirm the effect of increasing the volume of adipose tissue in breasts and buttocks of volunteers, as well as an improvement of the biomechanical properties thereof, such as firmness and elasticity.

An additional in vitro assay was performed to better understand the cellular pathways and processes that were modulated by RFE in the adipose cells. To this end, a comprehensive assessment of gene expression was conducted by transcriptome analysis. For this study 3T3-L1 pre-adipocytes were stimulated with RFE during their differentiation process into adipocytes, and at the end of the treatment, RNA was extracted. Next, samples were processed by RNA sequencing (RNA-Seq) and obtained data were studied by bioinformatic analysis. Hierarchical clustering analysis showed that RFE boosts the differentiation program of the adipocytes. Differential expression analysis revealed that RFE the significantly changed the expression of 5234 genes (2784 down and 2540 up-regulated) against differentiated control. Those changes mainly impacted on lipid metabolic pathways, for example those that favor fatty acid biosynthesis and the production of characteristic lipids of mature adipocytes. Other gene expression changes revealed that RFE favors the characteristic rounded shape of the adipocyte and the remodeling of the hypodermal extracellular matrix to allow adipose tissue growth and limit fibrosis.

### Rubus fruticosus fruit extract (RFE)

The biomass of interest is the fruit of blackberry, *Rubus fruticosus.* In particular, of special interest is the biomass from *Rubus fruticosus* dried triturated fruits.

Prior to the extraction process, the biomass can be subjected to fermentation or enzymatic treatments for the purposes of breaking cell walls, which can make any bioactives present in the biomass more readily available for extraction.

The term "extract" means any compound which has been extracted from the *Rubus fruticosus* fruit.

The term "biomass" refers to the *Rubus fruticosus* fruit.

In an embodiment, the RFE is a solution in the extracting solvent.

In an embodiment, the RFE is a dried extract, wherein the extracting solvent mixture is removed to obtain a substantially solid product.

Extraction for purposes of this invention means treatment of the biomass with a liquid or exposure of the biomass thereto. Compounds are retrieved from the solid biomass via solubilization in the liquid phase. In particular, compounds having bioactive properties are of special interest.

The extraction of compounds, such as botanical active ingredients, from vegetable matter is well known to the skilled in the art and is described, for example, in the book S.C. Mandal, V. Mandal and A.K. Das, Essentials of Botanical Extraction: Principles and Applications, Elsevier, San Diego, 2015.

Extraction processes are well-known to those having ordinary skill in the extract field (e.g., maceration, infusion, percolation, digestion, decoction, hot continuous extraction, counter current extraction, microwave assisted extraction, ultrasound extraction, supercritical fluid extraction, etc). Preferred extraction processes are maceration, decoction, infusion, hot continuous extraction, and combinations thereof.

Blackberry fruits are preferably dried prior to extraction.

The extraction process generally comprises the following steps:
a) contacting *Rubus fruticosus* fruits with the extraction solvent forming the extraction mixture,
b) extracting the *Rubus fruticosus* fruits with the extraction solvent at a temperature higher than room temperature, and optionally
c) macerating the extraction mixture at room temperature.

Usually, the process comprises an additional step, wherein the *Rubus fruticosus* fruits are separated from the extraction mixture.

The extraction solvent is usually selected from water, alcohols, polyalcohols, such as glycols and glycerol, oils, and mixtures thereof. Preferred solvents include water, ethanol, glycerol, 1,3-propanediol, propylene glycol, butylene glycol, and mixtures thereof; more preferably water, ethanol, glycerol, 1,3-propanediol, and mixtures thereof; yet more preferably water, ethanol, 1,3-propanediol, and mixtures thereof.

In an embodiment, the extraction can also be carried out in supercritical conditions, thus avoiding the use of liquid solvents. In this case fluids in a supercritical state are used, such as, for example, carbon dioxide, nitrous oxide, ammonia, n-heptane, n-butane or xenon.

The extraction temperature and duration of the extraction process is established on the basis of the extraction liquid that is used.

Usually, in the context of the invention, the extraction process is carried out at a temperature comprised between 70°C and 100°C, preferably between 80°C and 100°C.

Generally, in the context of the invention, the extraction is continued for a period of time comprised between 2 and 72 hours, preferably from 2 to 48 hours, more preferably from 2 to 24 hours.

In an embodiment, the extraction process combines the extraction at a temperature comprised between 70°C and 100°C, preferably between 80°C and 100°C, and a maceration process at room temperature. In an embodiment, the hot extraction is carried out for not more than 12 hours, preferably not more than 8 hours, more preferably not more than 6 hours, and the maceration process is carried out for at least 10 hours, preferably from 10 to 15 hours, and more preferably at least 16 hours.

In an embodiment, the extraction solvent may additionally contain further additives, selected from co-solvents, solubilizers, antioxidants, preservatives, pH adjusters, rheology modifiers, and mixtures thereof. In an embodiment, said further additive may be added at a later step.

In an embodiment, the biomass (*Rubus fruticosus* fruit) is put into intimate contact with a liquid, the extraction solvent, to form a slurry. The content of the extraction solvent is comprised usually between about 50 wt% and about 99.5 wt%, preferably between 60 wt% and 99.4 wt%, more preferably between 65 wt% and 99.3 wt%, more preferably between 70 wt% and 99.2 wt%, and yet more preferably between about 75 wt% and 99 wt%.

In an embodiment, the extraction is carried out using a combination of ethanol and water as extraction solvent. In an embodiment, the ratio of ethanol to water is comprised between 5:1 and 1:1, preferably between 4:1 and 2:1, and more preferably is about 2.5:1. The extraction is preferably carried out at a reflux temperature.

In an embodiment, the extraction is carried out using a combination of 1,3-propanediol and water as extraction solvent. In an embodiment, the ratio of 1,3-propanediol to water is comprised between 10:1 and 3:2, preferably between 5:1 and 3:1, and more preferably is about 4:1. The extraction is preferably carried out at a temperature comprised between 80°C and 100°C. In an embodiment, the extraction process using 1,3-propanediol and water mixtures combines the extraction at a temperature comprised between 70°C and 100°C, preferably between 80°C and 100°C, and a maceration process at room temperature. In an embodiment, the hot extraction is carried out for not more than 12 hours, preferably not more than 8 hours, more preferably not more than 6 hours, and the maceration process is carried out for at least 10 hours, preferably from 10 to 15 hours, and more preferably at least 16 hours.

In an embodiment, the extraction is carried out using 1,3-propanediol and water as solvent, in the presence of an organic acid, preferably citric acid.

In an embodiment, the extraction is carried out at a pH adjusted between 3.0 and 6.2, preferably between 4.0 and 5.0, and more preferably between 4.3 and 4.6.

To improve the extraction yield, the extraction can be a multiple extraction process, wherein the extraction process is repeated two or three times, or more, using fresh extraction solvent each time. The successive extracts are collected together and further processed.

Al the end of the extraction, the biomass, *Rubus fruticosus* fruits, is separated from the extraction mixture by processes well-known to those having ordinary skill in the extract field (e. g., decantation, filtration, press or vacuum filtration, centrifugation, etc).

The extract can then be stored and used in liquid form, or subject to further processing techniques (e.g., heating, cooling, evaporation, concentration, pasteurization, spray-drying, lyophilization, fractionation, etc.).

In an embodiment, the solvent used in the extraction is evaporated, preferably under vacuum, to yield a solid RFE.

In an embodiment, the solvent used in the extraction is partially evaporated to yield a solution of the RFE.

The extract obtained with this process can contain one or more metabolites of *Rubus fruticosus,* including phenolic substances (such as anthocyanins, procyanidins, flavonols, flavan-3-ols, phenolic acid derivatives and ellagitannins), sugars (such as glucose and fructose), organic acids (such as malic, citric, isocitric and ascorbic acids).

Blackberry anthocyanins identified include cyanidin 3-glucoside, cyanidin 3-galactoside, cyanidin 3-xyloside, cyanidin 3-dioxalyl-glucoside, cyanidin 3-rutinoside, cyanidin 3-sophoroside, cyanidin 3-glucosylrutinoside, cyanidin 3-arabinoside, malvidin 3-arabinoside, perlargonidin 3-glucoside, cyanidin 3-(3-malonyl) glucoside, and cyanidin 3-(6-malonyl) glucoside.

Blackberry flavonols include quercetin 3-galactoside, quercetin 3-glucoside, quercetin 3-rutinoside, quercetin 3-xylosylglucuronide, quercetin 3-glucuronide, kaempferol 3-glucuronide, kaempferol 3-glucoside, kaempferol 3-galactoside, kaempferol 3-xylosylglucuronide and myricetin and myricetin glycosides.

Blackberry ellagitannins include sanguiin compounds, lambertianins, pedunculagin, galloyl-bis-HHDP glucose, castalagin and ellagic acid.

Blackberry phenolic acid derivatives include esters and glycosides of *p-*hydrobenzoic, protocatechuic, gallic, vanillic, salicylic, syringic, gentisic, caffeic, *m-*coumaric, *p*-coumaric, and ferulic acids.

In addition, the RFE can contain lower amounts of lignans, vitamins, minerals and trace elements.

The RFE for the use of the present invention comprises usually between 3 wt% and 7 wt%, preferably between 4 et% and 6 wt%, more preferably about 5 wt% of total phenolic content over the total solid content of the RFE.

The total phenolic content can be determined according to any analytical method well-known to the skilled person in the art, as disclosed, for example, in AOAC Official Methods Analysis, 1995, 952.03, Chapter 26, page 16, or an adaptation thereof.

### Cosmetic compositions

In an embodiment of the use of the invention, the RFE is present in a cosmetic composition.

In an embodiment, the RFE is incorporated as a solid to said cosmetic composition.

In an embodiment, the RFE is incorporated as a solution to said cosmetic composition.

In an embodiment, the RFE is incorporated to said composition in combination with additives, such as additional solvents or solubilizers, antioxidants, preservatives, pH adjusters and rheology modifiers.

The amount of RFE in a cosmetic composition for the use of the invention depends on the desired effect and must be in a sufficient quantity for increasing adipocyte mass in the adipose tissue, i.e., to stimulate the expansion and/or the formation of the subcutaneous adipose tissue.

In an embodiment the cosmetic composition comprises the RFE and a cosmetically acceptable component.

As used herein, "cosmetically-acceptable" means that the product(s) or compound(s) which the term describes are suitable for use in contact with tissues (e.g., the skin) without undue toxicity, incompatibility, instability, irritation, allergic response, and the like. This term is not intended to limit the ingredient/product, which it describes to use solely as a cosmetic

In an embodiment, the cosmetic composition contains:
a) between 0.001 wt% and 1 wt% of the RFE, as solid, and
b) between 99 wt% and 99.999 wt% of a cosmetically acceptable component,
where the percentages of the components are adjusted so that the balance is 100%.

The composition preferably contains between 0.005 wt% and 0.75 wt% of the RFE, as solid, more preferably between 0.008 wt% and 0.5 wt%, more preferably between 0.01 wt% and 0.1 wt%, and yet more preferably between 0.02 wt% and 0.05 wt%.

In the case of using a solution of the RFE, the skilled person in the art can determine routinely the amount of solution needed to obtain the required amount of solid RFE.

The cosmetic composition comprises a cosmetically acceptable component, which in the context of the invention, is selected from a cosmetically acceptable vehicle, a cosmetic ingredient, and mixtures thereof.

A cosmetically acceptable vehicle (or carrier) is a vehicle in which the cosmetic ingredients are dissolved, emulsified, dispersed, or suspended. Said vehicle is selected from among water, a water-miscible non-aqueous vehicle, such as ethanol or isopropanol, and a water-immiscible non-aqueous vehicle, such as, for example, vegetable oil, fatty esters, medium chain triglycerides, or alkanes. Preferably the cosmetic composition includes water as a vehicle.

In an embodiment, the cosmetic composition contains at least one additional cosmetic ingredient, such as, for example, cosmetic active ingredients, auxiliary products and additional additives.

In an embodiment, the additional cosmetic ingredient can be selected from: skin soothing and healing agents, skin anti-aging agents, skin moisturizing agents, anti-wrinkle agents, anti-atrophy agents, skin smoothing agents, antibacterial agents, antifungal agents, antimicrobial agents, anti-inflammatory agents, anti-pruriginous agents, external anaesthetic agents, , keratolytic agents, free radicals scavengers, anti-seborrheic agents, antidandruff agents, the agents modulating the differentiation, proliferation or pigmentation of the skin and agents accelerating penetration, desquamating agents, depigmenting or propigmenting agents, tightening agents, agents stimulating the synthesis of dermal or epidermal macro molecules and/or preventing their degradation, agents stimulating the proliferation of fibroblasts and/or keratinocytes or stimulating the differentiation of keratinocytes, muscle relaxants; antipollution and/or anti- free radical agents, slimming agents, anticellulite agents, agents acting on the microcirculation, agents acting on the energy metabolism of the cells, cleaning agents, hair conditioning agents, hair styling agents, hair growth promoters, sunscreen and/or sunblock compounds, make-up agents, detergents, emulsifiers, emollients, antiseptic agents, deodorant actives, dermatologically acceptable carriers, surfactants, abrasives, absorbents, colorants, essential oils, cosmetic astringents, anti-acne agents, anti-caking agents, anti-foaming agents, antioxidants, binders, biological additives, enzymes, enzymatic inhibitors, enzyme-inducing agents, coenzymes, plant extracts, plant derivatives, plant tissue extracts, plant seed extracts, plant oils, botanicals, botanical extracts, ceramides, peptides, buffering agents, bulking agents, chelating agents, chemical additives, colorants, cosmetic biocides, denaturants, astringents compounds, film formers, quaternary derivatives, agents increasing the substantivity, opacifying agents, pH adjusters, pH regulators, propellants, reducing agents, sequestrants, skin bleaching and lightening agents, skin tanning agents, skin-conditioning agents, skin soothing and/or healing agents, skin treating agents, thickeners, vitamins and derivatives thereof, peeling agents, moisturizing agents, lignans, UV absorbers, suspending agents, viscosity modifiers, pigments, dyes, non-volatile solvents, diluents, pearlescent aids, foam boosters, water-soluble sunscreen, antiperspirant, depilatory, fat soluble sunscreen, skin restructuring agent, emollient, fillers, minerals, anti-mycobacterial agents, anti-allergenic agents, anti-irritants, immune system boosting agents, immune system suppressing agents, insect repellents, lubricants, staining agents, hypopigmenting agents, preservatives, photostabilizing agents and their mixture.

In an embodiment, the additional cosmetic ingredient is preferably selected from: surfactants (emulsifiers), lipid compounds, emollients, factors of consistency, thickening agents, stabilizers, hydrotropes, preserving agents, essences, colorants, silicone compounds, fats, waxes, lecithins, phospholipids, UV sun protection factors, film-forming agents, self-tanners, firming agents, cosmetic actives, and mixtures thereof.

In a preferred embodiment, the cosmetic composition contains an additional cosmetic ingredient selected from the group consisting of surfactants (emulsifiers), lipid, emollient compounds, consistency factors, thickening agents, hydrotropes, preservatives, cosmetic actives, and mixtures thereof.

The physical form of the cosmetic composition for the use according to the invention is not important. Said composition may be made into a wide variety of product types that include but are not limited to solid and liquid compositions such as solutions, lotions, creams, gels, sticks, sprays, ointments, cleansing liquid washes and solid bars, shampoos, pastes, powders, foams, mousses, milks, emulsions, dispersions, suspensions, or wipes.

The topical compositions useful in the present invention may be formulated as solutions. Solutions may preferably include an aqueous solvent (e.g., from about 75 wt% to about 95 wt% or from about 75 wt% to about 85 wt% of a cosmetically acceptable aqueous solvent). More preferably, such compositions may contain about 30 wt% solvent, although this may vary dependent upon the formulation. Such solvents may include ethanol, propylene glycol, polyethylene glycol, mixtures thereof and the like. In an embodiment, the topical composition useful in the present invention may be formulated as a solution containing an emollient. Such composition preferably contains from about 2 wt% to about 50 wt% of an emollient. As used herein, "emollients" refer to materials used for the prevention or relief of dryness, as well as for the protection of the skin.

A lotion may be made from a solution. Lotions typically contain from about 1 wt% to about 20 wt% of an emollient and from about 50 wt% to about 90 wt% of water.

Another type of product may be a cream. A cream typically comprises from about 5 wt% to about 50 wt% of an emollient and from about 45 wt% to about 85 wt% of water.

Another type of product may be an ointment. An ointment may be constituted of a simple base of vegetable oils or semi-solid hydrocarbons. An ointment may contain from about 2 wt% to about 100 wt% of an emollient, and from about 0.1 wt% to about 5 wt% of a thickening agent.

The topical compositions useful in the present invention may also be preferably formulated as emulsions. In an embodiment, the cosmetic composition contains water and a lipophilic phase and is presented in the form of emulsion or dispersion, such as, for example, the oil-in-water (O/W), water-in-oil (W/O) type, multiple emulsion (W/O/W), or PIT-type emulsion, or microemulsion. If the carrier is an emulsion, from about 1 wt% to about 10 wt% of the carrier should be made up one or more emulsifiers. Emulsifiers may be nonionic, anionic, cationic, or amphoteric.

The topical composition useful in the present invention may be formulated as a gel. Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as vegetable oils, esters) include, but are not limited to, waxes, modified silicas, cellulose derivatives, polyamides, polyurethanes, and L-glutamic acid derivatives. Such gels typically comprise between about 0.1 wt% and 20 wt% of such gelling agents.

The topical composition useful in the present invention may also be formulated into a solid formulation (e.g., a wax-based stick, mascara, soap bar composition, powder, a wipe containing powder, or cosmetotextile).

In a preferred embodiment, the cosmetic composition comprises:
a) between 0.001 wt% and 1 wt% of the RFE, as solid,
b) between 5 wt% and 50 wt% of at least one additional cosmetic ingredient, and
c) between 49 wt% and 94 wt% of a cosmetically acceptable vehicle,
where the percentages of the components are adjusted so that the balance is 100%.

In an embodiment, the content of the additional cosmetic ingredient is preferably between 10 wt% and 45 wt%, more preferably between 15 wt% and 35 wt%, and even more preferably between 20 wt% and 25 wt%.

In an embodiment the content of the vehicle is preferably between 50 wt% and 90 wt%, more preferably between 55 wt% and 87 wt%, and even more preferably between 65 wt% and 85 wt%.

The cosmetic composition useful for the use of the present invention are generally prepared by conventional methods such as are known in the art of making topical compositions. Such methods typically can involve mixing of the ingredients in one or more steps to a relatively uniform state, with or without heating, cooling, application of vacuum, and the like.

### Surfactants (emulsifiers)

In an embodiment, the cosmetic composition can include surfactants (emulsifiers) to facilitate solution, emulsion, dispersion, or suspension of cosmetic components.

Surfactants can be anionic, non-ionic, cationic and/or amphoteric.

The content of surfactants is usually comprised between 1 wt% and 30 wt%, preferably between 2 wt% and 20 wt%, more preferably between 3 wt% and 10 wt%, and still more preferably between 4 wt% and 8 wt%.

Typical examples of anionic surfactants are, for example, soaps, sulfonated alkanes, sulfonated olefins, alkyl sulfates, fatty alcohol ether sulfates, glycerol ether sulfates, fatty acid ether sulfates, mono- and dialkyl sulfosuccinates, mono- and dialkyl sulfosuccinamates, ether carboxylic acids and their salts, fatty acid isethionates, fatty acid sarcosinates, fatty acid taurides, *N*-acylamino acids.

Typical examples of non-ionic surfactants are, for example, polyalkoxylated fatty alcohols, polyalkoxylated fatty acids, polyalkoxylated fatty acid amides, polyalkoxylated fatty amines, alkoxylated triglycerides, mixed ethers, alkylpolyglycosides, *N*-alkyl sorbitan esters, fatty acid esters polyethoxylated sorbitan, and amine oxides.

Typical examples of cationic surfactants are, for example, quaternary ammonium compounds, and quaternized salts of esters of trialkanolamines and fatty acids, for example Esterquats.

Typical examples of amphoteric surfactants are, for example, alkylbetaines, alkylamidobetaines, aminopropionates, aminoglycinates, imidazolinium betaines and sulfobetaines.

The aforementioned surfactants are exclusively known compounds, the structure and preparation of which are well known to those skilled in the art, and can be found, for example, in X. Domingo's book, A guide to the surfactants world, Edicions Proa, Barcelona, 1995.

### Lipid components and emollients

The cosmetic composition usually contains additional lipid compounds and emollients to optimize their organoleptic and dermatological properties.

The lipid and emollient compounds are usually contained in a total amount of between 1 wt% and 50 wt%, preferably between 5 wt% and 25 wt%, and more preferably between 5 wt% and 15 wt%.

Suitable lipid compounds are Guerbet alcohols based on fatty alcohols with 6 to 18 carbon atoms, preferably with 8 to 10 carbon atoms (such as the product Eutanol^{®} G from the BASF company), linear fatty acid esters C₆₋₂₂ with linear C₆₋₂₂ alcohols, C₆₋₁₃ branched carboxylic acid esters with C₆₋₂₂ linear alcohols, such as myristyl myristate, carbon palmitate myristyl, myristyl stearate, behenyl stearate, behenyl isostearate, behenyl oleate, behenyl behenate, behenyl erucate, or erucyl myristate.

Furthermore, C₆₋₂₂ linear fatty acid esters with branched alcohols are suitable, especially 2-ethylhexanol (for example the product Cetiol^{®} 868 from the BASF company); C₁₈₋₃₈ alkylhydroxycarboxylic acid esters with linear or branched C₆₋₂₂ fatty alcohols; linear and/or branched fatty acid esters with polyhydric alcohols, and/or Guerbet alcohols; triglycerides based on fatty acids with 6 to 10 carbon atoms (for example the product Mirytol^{®} 318 from the company BASF); liquid mixtures of mono/di/triglycerides based on fatty acids with 6 to 18 carbon atoms; esters of fatty alcohols with 6 to 22 carbon atoms, and/or Guerbet alcohols with aromatic carboxylic acids, especially benzoic acid; esters of dicarboxylic acids with 2 to 12 carbon atoms with linear or branched alcohols of 1 to 22 carbon atoms, or polyols with 2 to 10 carbon atoms and 2 to 6 hydroxyl groups; vegetable oils; branched primary alcohols; substituted cyclohexanes; linear and branched C₆₋₂₂ fatty alcohol carbonates, such as, for example, dicaprylyl carbonates (for example the product Cetiol^{®} CC from the BASF company); symmetric or asymmetric linear or branched dialkyl ethers with 6 to 22 carbon atoms per alkyl group, such as dicaprylyl ethers (for example the product Cetiol^{®} OE from the BASF company); aliphatic or naphthenic hydrocarbons, such as squalane, squalene or dialkylcyclohexanes, and mixtures thereof.

### Consistency factors and thickeners

Consistency factors and thickeners are generally used in the cosmetic composition to adjust the viscosity and rheological behaviour.

Among the consistency factors come into consideration, firstly, fatty alcohols with a chain of 12 to 22 carbon atoms, and preferably of 16 to 18 carbon atoms, and furthermore partial glycerides, fatty acids or hydroxylated fatty acids.

Suitable thickening agents are, for example, hydrophilic silicic anhydride (such as the Aerosil^{®} products from the Evonik company); polysaccharides, especially xanthan gum, guar gum, agar-agar, alginates and tyloses, carboxymethylcellulose and hydroxyethylcellulose, as well as polyethylene glycol mono- and higher molecular weight fatty acid diesters; polyacrylates (for example Carbopol^{®} and Pemulen^{®} types from the Lubrizol company; Synthalene^{®} from the Sigma company, Keltrol^{®} types from the CP Kelco company; Sepigel^{®} and Simulgel^{®} types from the Seppic company; Salcare^{®} types from the company Allied Colloids company), polyacrylamides, polyvinyl alcohol, and polyvinylpyrrolidone.

### Hydrotropes

In order to improve the flowability of the cosmetic composition, it is also possible to use hydrotropes, such as, for example, ethanol, isopropyl alcohol, or polyols. The polyols which come into consideration here preferably have from 2 to 15 carbon atoms and at least two hydroxyl groups. The polyols can also contain other functional groups, especially amino groups, or be modified with nitrogen. Typical examples are glycerol; alkylene glycols, such as, for example, ethylene glycol, diethylene glycol, propylene glycol, butylene glycol, hexylene glycol, as well as polyethylene glycols with an average molecular weight of 100 to 1,000 daltons; technical oligoglycerol mixtures with a degree of self-condensation of 1.5 to 10, for example technical diglycerol mixtures with a diglycerol content of 40 wt% to 50 wt%; methylol compounds, such as especially trimethylolmethane, trimethylolpropane, trimethylolbutane, pentaerythritol and dipentaerythritol; alkylglycosides with a chain of 1 to 8 carbon atoms in the alkyl moiety; C₅₋₁₂ sugar alcohols, such as sorbitol or mannitol; sugars with 5 to 12 carbon atoms, such as glucose or sucrose; amino sugars, such as glucamine; dialcoholamines, such as diethanolamine or 2-amino-1,3-propanediol.

In an embodiment, glycerol, propylene glycol, and their mixtures are used as hydrotrope in the cosmetic composition.

### Preservative agents

Suitable preservatives to be used in the cosmetic composition include, by way of example, phenoxyethanol, 3-(4-chlorophenoxy)-1,2-propanediol (chlorphenesin), pentanediol or sorbic acid, and the classes of additional substances indicated in Annex V of the Regulation (EC) No 1223/2009 of the European Parliament and of the Council.

### Cosmetic active ingredients

The cosmetic composition for use in the present invention can also contain at least one complementary cosmetic active chosen among the active ingredients stimulating the lipogenesis and/or adipogenesis, strengthening active ingredients, active ingredients acting on skin's tonicity, active ingredients having curving effects, vasodilator active ingredients, active ingredients favouring microcirculation, and their mixture.

In an embodiment, suitable cosmetic actives are selected from extracts obtained from: hops, *Kigelia africana,* sage, soya, *Sabal serrulata,* damiana, de dong quai, dandelion, cumin, liquorice, *Cnicus benedictus,* red elm, raspberry bush, lupin, fennel, pepper, malt, mourere, Kombucha, clover, *Anemarrhena asphodeloides,* alchemille, and mixtures thereof.

### Method

An aspect of the invention is a cosmetic method for increasing adipocytic mass in adipose tissue comprising the step of topically applying to skin an RFE or a cosmetic composition comprising an RFE.

In an embodiment, the RFE is obtained according to the method disclosed above.

In an embodiment, the method of the invention comprises the step of topically applying to the bust, and/or the low neckline, and/or the buttocks, and/or the face, and/or the lips, and/or the cheeks, and/or the eyelids, and/or the hands, the RFE or a cosmetic composition comprising the RFE.

In an embodiment, the RFE or the cosmetic composition comprising the RFE is applied topically to the desired area of the body at least once daily, preferably between 1 to 3 times daily, and more preferably, 1 to 5 times daily.

In an embodiment, the RFE is applied topically to the desired area of the body for at least 2 weeks, preferably at least 4 weeks, more preferably at least 8 weeks, and more preferably at least 12 weeks.

The invention comprises the following embodiments:
1.- Cosmetic and dermatological use of a *Rubus fruticosus* fruit extract (RFE) for increasing adipocytic mass by topical application.
2.- Cosmetic and dermatological use according to embodiment 1, characterized in that the RFE is for increasing adipocytic mass by stimulating adipogenesis and/or lipogenesis, and reducing lipolysis in adipose tissue.
3.- Cosmetic and dermatological use according to embodiment 2, characterized in that the RFE is for stimulating adipogenesis and reducing lipolysis in adipose tissue.
4.- Cosmetic and dermatological use according to embodiment 2, characterized in that the RFE is for stimulating lipogenesis and reducing lipolysis in adipose tissue.
5.- Cosmetic and dermatological use according to any one of embodiments 1 to 4, characterized in that the increase of adipocytic mass has a volume increasing effect of the adipose tissue.
6.- Cosmetic and dermatological use according to any one of embodiments 1 to 5, characterized in that the topical application is to the breasts, and/or the low neckline, and/or the buttocks, and/or the face, and/or the lips, and/or the cheeks, and/or the eyelids, and/or the hands of a person in need.
7.- Cosmetic and dermatological use according to any one of embodiments 1 to 6, characterized in that the RFE is obtained from dried and triturated fruits.
8.- Cosmetic and dermatological use according to any one of embodiments 1 to 7, characterized in that the RFE is a solution in the extracting solvent.
9.- Cosmetic and dermatological use according to any one of embodiments 1 to 8, characterized in that the RFE is a dried extract.
10.- Cosmetic and dermatological use according to any one of embodiments 1 to 9, characterized in that the RFE is obtained by an extraction process comprising the following steps:
   a) contacting *Rubus fruticosus* fruits with the extraction solvent forming the extraction mixture,
   b) extracting the *Rubus fruticosus* fruits with the extraction solvent at a temperature higher than room temperature, and optionally
   c) macerating the extraction mixture at room temperature.
11.- Cosmetic and dermatological use according to embodiment 10, characterized in that the extraction solvent is selected from water, alcohols, polyalcohols, oils, and mixtures thereof.
12.- Cosmetic and dermatological use according to embodiment 11, characterized in that the extraction solvent is selected from water, ethanol, glycerol, 1,3-propanediol, propylene glycol, butylene glycol, and mixtures thereof; preferably water, ethanol, glycerol, 1,3-propanediol, and mixtures thereof; more preferably water, ethanol, 1,3-propanediol, and mixtures thereof.
13.- Cosmetic and dermatological use according to embodiment 12, characterized in that the extraction is carried our using a combination of ethanol and water as extraction solvent.
14.- Cosmetic and dermatological use according to embodiment 13, characterized in that the ratio of ethanol to water is comprised between 5:1 and 1:1, preferably between 4:1 and 2:1, and more preferably is about 2.5:1.
15.- Cosmetic and dermatological use according to embodiment 12, characterized in that the extraction is carried out using a combination of 1,3-propanediol and water as extraction solvent.
16.- Cosmetic and dermatological use according to embodiment 15, characterized in that the ratio of 1,3-propanediol to water is comprised between 10:1 and 3:2, preferably between 5:1 and 3:1, and more preferably is about 4:1.
17.- Cosmetic and dermatological use according to any one of embodiments 10 to 16, characterized in that the extraction process is carried out at a temperature comprised between 70°C and 100°C, preferably between 80°C and 100°C.
18.- Cosmetic and dermatological use according to any one of embodiments 10 to 17, characterized in that the extraction process is continued for a period of time comprised between 2 and 72 hours, preferably from 2 to 48 hours, more preferably from 2 to 24 hours.
19.- Cosmetic and dermatological use according to any one of embodiments 10 to 18, characterized in that the extraction process combines the extraction at a temperature comprised between 70°C and 100°C, preferably between 80°C and 100°C, and a maceration process at room temperature.
20.- Cosmetic and dermatological use according to embodiment 19, characterized in that the extraction process is carried out for not more than 12 hours, preferably not more than 8 hours, more preferably not more than 6 hours, and the maceration process is carried out for at least 10 hours, preferably from 10 to 15 hours, and more preferably at least 16 hours.
21.- Cosmetic and dermatological use according to any one of embodiments 10 to 20, characterized in that the extraction solvent additionally contains further additives selected from co-solvents, solubilizers, antioxidants, preservatives, pH adjusters, rheology modifiers, and mixtures thereof.
22.- Cosmetic and dermatological use according to any one of embodiments 10 to 21, characterized in that the content of the extraction solvent is comprised between about 50 wt% and about 99.5 wt%, preferably between 60 wt% and 99.4 wt%, more preferably between 65 wt% and 99.3 wt%, more preferably between 70 wt% and 99.2 wt%, and yet more preferably between about 75 wt% and 99 wt%.
23.- Cosmetic and dermatological use according to any one of embodiments 1 to 22, characterized in that the RFE is present in a cosmetic composition.
24.- Cosmetic and dermatological use according to embodiment 23, characterized in that the cosmetic composition comprises the RFE and a cosmetically acceptable component.
25.- Cosmetic and dermatological use according to embodiment 24, characterized in that the cosmetic composition comprises between 0.001 wt% and 1 wt% of the RFE, as solid, and between 99 wt% and 99.999 wt% of a cosmetically acceptable component, where the percentages of the components are adjusted so that the balance is 100%.
26.- Cosmetic and dermatological use according to embodiment 24 or 25, characterized in that the cosmetically acceptable component is selected from a cosmetically acceptable vehicle, a cosmetic ingredient, and mixtures thereof.
27.- Cosmetic and dermatological use according to any one of embodiments 24 to 26, characterized in that the cosmetic acceptable ingredient is selected from: surfactants (emulsifiers), lipid compounds, emollients, factors of consistency, thickening agents, stabilizers, hydrotropes, preserving agents, essences, colorants, silicone compounds, fats, waxes, lecithins, phospholipids, UV sun protection factors, film-forming agents, self-tanners, firming agents, cosmetic actives, and mixtures thereof.
28.- Cosmetic and dermatological use according to any one of embodiments 24 to 27, characterized in that the cosmetic composition is in form of solutions, lotions, creams, gels, sticks, sprays, ointments, cleansing liquid washes and solid bars, shampoos, pastes, powders, foams, mousses, milks, emulsions, dispersions, suspensions, or wipes.
29.- Cosmetic and dermatological use according to embodiment 26, characterized in that the cosmetic composition comprises:
   a) between 0.001 wt% and 1 wt% of RFE, as solid,
   b) between 5 wt% and 50 wt% of at least one additional cosmetic ingredient, preferably between 10 wt% and 45 wt%, more preferably between 15 wt% and 35 wt%, and even more preferably between 20 wt% and 25 wt%, and
   c) between 49 wt% and 94 wt% of a cosmetically acceptable vehicle, preferably between 50 wt% and 90 wt%, more preferably between 55 wt% and 87 wt%, and even more preferably between 65 wt% and 85 wt%,
   where the percentages of the components are adjusted so that the balance is 100%.
30.- Cosmetic and dermatological use according to embodiment 27, characterized in that the cosmetic active is selected from extracts obtained from: hops, *Kigelia africana,* sage, soya, *Sabal serrulata,* damiana, de dong quai, dandelion, cumin, liquorice, *Cnicus benedictus,* red elm, raspberry bush, lupin, fennel, pepper, malt, mourere, Kombucha, clover, *Anemarrhena asphodeloides,* alchemille, and mixtures thereof.
31.- Cosmetic and dermatological method for increasing adipocytic mass in adipose tissue comprising the step of topically applying to skin an RFE or a cosmetic composition comprising an RFE.
32.- Cosmetic and dermatological method according to embodiment 31, characterized in that it comprises the step of topically applying to the bust, and/or the low neckline, and/or the buttocks, and/or the face, and/or the lips, and/or the cheeks, and/or the eyelids, and/or the hands, the RFE or a cosmetic composition comprising the RFE.
33.- Cosmetic and dermatological method according to embodiment 31 or 32, characterized in the RFE or the cosmetic composition comprising the RFE is applied topically to the desired area of the body at least once daily, preferably between 1 to 3 times daily, and more preferably, 1 to 5 times daily.
34.- Cosmetic and dermatological method according to any one of embodiments 31 to 33, characterized in that the RFE or the cosmetic composition comprising the RFE is applied topically to the desired area of the body for at least 2 weeks, preferably at least 4 weeks, more preferably at least 8 weeks, and more preferably at least 12 weeks.

In the following examples, different embodiments of the invention are provided for illustrative purposes that are understood to be non-limiting.

### Examples

Methods and media compositions used in this section are available to the skilled person in the art in, for example, 3T3-L1 Cell Care Manual. Maintenance and Differentiation of 3T3-L1 Preadipocytes to Adipocytes, Instruction Manual ZBM0009.06 (ZenBio, Inc.).

Total phenolic content was determined according to a method based on the method disclosed in AOAC Official Methods Analysis, 1995, 952.03, Chapter 26, page 16.

The geographic origin of the *Rubus fruticosus* fruits used in the *in vitro* and *in vivo* tests is Chile and Serbia, respectively.

The following abbreviations are used in the Examples:
- DMEM:F12: Dubelco's Modified Eagle Medium/Nutrient Mixture F-12
- NCS: Newborn calf serum
- FBS: Fetal bovine serum
- IBMX: Isobutyl methylxantine

### Example 1: Preparation of a Rubus fruticosus fruit extract

*Rubus fruticosus* dried triturated fruits were placed in a reflux system along with a mixture of ethanol and water, in a ratio of ethanol:water comprised between 5:1 and 1:1. In the extraction system, the content of the solvent was comprised between 50 wt% and 99.5 wt%. The mixture was refluxed for 3 h, the liquid was decanted and to the remaining biomass additional ethanol and water, in the same proportions as above, were added. This mixture was refluxed again for 3 h. The liquid was decanted and mixed with the liquid from the first reflux. Solvents were then evaporated under vacuum to yield a soft solid extract (52%). This extract contained 5.35% Total Phenolic Content (TPC).

### Example 2: Preparation of a Rubus fruticosus fruit extract

To a solvent mixture of 1,3-propanediol and water, in a ratio of 1,3-propanediol:water comprised between 10:1 and 3:2, citric acid was added. The pH was adjusted to 4.3-4.6 with sodium hydroxide (20% solution) and the mixture was heated to 90-100°C. *Rubus fruticosus* dried triturated fruits were added to the mixture, wherein the content of the solvent was comprised between 50 wt% and 99.5 wt%. The temperature was maintained at 80°C for 2 h. The mixture was allowed to cool to room temperature, and it was then macerated for a period comprised between 6 and 24 h. The spent biomass was pressed, and the liquid was recovered. The liquid was then clarified by filtration with the aid of diatomaceous earth. The resulting product with a dry residue of 2.88% contained 0.113% of Total Phenolic Content (TPC).

### Example 3: Adipogenesis and lipogenesis assessment by Oil Red staining

3T3-L1 pre-adipocytes were sowed in 2 ml of growth medium in 6 wells plates and were cultivated for 4 days until total confluence.

Growth medium consists of DMEM, Penicillin/streptomycin (110U/ml / 100µl) 1%, NCS 10%. 48h later, the growth medium was replaced by differentiation medium DM1, together with 0.625 mg/ml of the soft solid RFE, obtained according to the process of Example 1. DM1 consists of DMEM:F12, Penicillin/streptomycin (110U/ml / 100µl) 1%, FBS 10%, IBMX0.5mM, Dexamethasone 1µM, Insulin 10 µM, Rosiglitazone 2 µM. After 48 hours, DM1 was replaced by differentiation medium DM2, including again the active. DM2 consists of DMEM:F12, Penicillin/streptomycin (110U/ml / 100µl) 1%, FBS 10%, Insulin 10 µM.

2 days later, samples were collected to assess the amount of lipids by using Oil-Red O staining (Sigma, ref. 01391). This is a neutral lipid-specific dye that allows visualizing triglycerides under the microscope, but also their relative quantification by measurement of absorbance at 500 nm. The greater the absorbance is, the higher the amount of lipids in the sample. Results were expressed as percentage of the variation of the absorbance, relative to Differentiated Control.

The study included 3 independent experiments with 9 replicates.

Scheme 1 shows the design of the experiment:

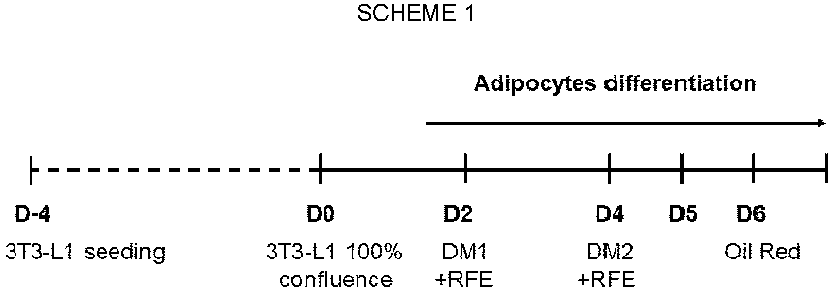

As can be observed in Figure 1, cells that were stimulated with DM1+DM2 (Control D) accumulated more than double of triglycerides than the non-differentiated cells (Control ND), thus confirming the induction of adipogenesis and lipogenesis process. In addition, when these differentiating cells received RFE treatment, the lipid content was significantly increased by 29%.

The confocal microscopy allowed to illustrate and visualize the adipogenic/lipogenic effect achieved by RFE, wherein lipids accumulated significantly in the adipocytes treated using RFE.

Preliminary results of *in vivo* tests confirmed the effect of increasing the volume of adipose tissue in breasts and buttocks of voluntary subjects of a formulation comprising the RFE (prepared according to a process substantially analogous to Example 2) in comparison to a placebo composition without said extract. The application of the product was performed twice per day for 12 weeks. The RFE was well tolerated by all the volunteers. Bust and buttocks were assessed for size and lifting effect (bust volume and distances; buttocks volume, perimeter and distances) using centimetric tape, VECTRA^{®}-XT 3D imaging system (Canfield Scientific) and biomechanical properties, such as firmness and elasticity using a cutometer (for example, CUTOMETER^{®} DUAL MPA 580, Courage + Khazaka Electronic).

### Example 4: Lipolysis assessment by glycerol measurement

3T3-L1 pre-adipocytes were sowed in 2 ml of growth medium in 6 wells plates and were cultivated for 4 days until total confluence. 48h later, the growth medium was replaced by differentiation medium DM1. After 48 hours, DM1 was replaced by differentiation medium DM2. The following day, 0.625 mg/ml of the soft solid RFE, obtained according to the process of Example 1 was added to the cell culture and adipocytes were stimulated with the active during 24h. After this time, lipolysis was determined with a commercial glycerol assay kit (Sigma, ref. 117). Absorbance was measured at 570 nm. Glycerol content was expressed as µg/mg protein. Protein was quantified with BCA assay kit (Pierce, ref. 23227). Results were expressed as percentage of the variation of the glycerol content, relative to Differentiated Control.

The study included 3 independent experiments with 9 replicates.

Scheme 2 shows the design of the experiment:

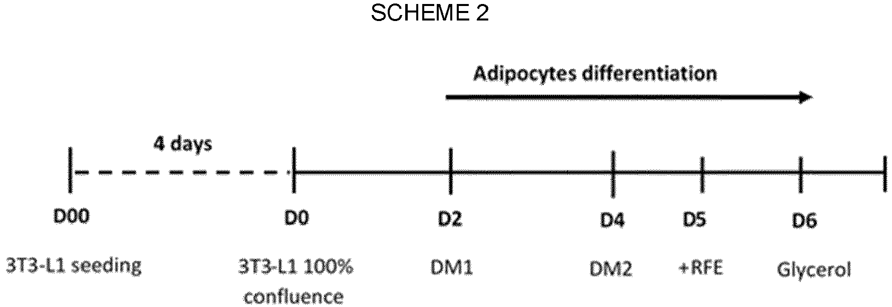

In Figure 2, the results of lipolysis assessment are shown. It can be observed that cells stimulated with DM1+DM2 (Control D) showed a 5-fold increase glycerol release, as compared to the non-differentiated cells (Control ND), thus confirming the normal metabolic activity of mature adipocytes. However, when these differentiated cells received RFE treatment, the glycerol release was significantly decreased by 51%, thus revealing RFE capacity to prevent triglyceride hydrolysis.

### Example 5: Composition

Using a *Rubus fruticosus* fruit extract obtained according to the process disclosed in Example 2, the composition shown in Table I was prepared:

**TABLE I**

| Component | wt% |
|---|---|
| Alcohol denat. | 10.00 |
| Dicaprylyl ether | 2.00 |
| Acrylates/C10-30 Alkyl Acrylate Crosspolymer | 0.75 |
| Polymethyl methacrylate, sodium acrylate/Sodium Acryloyldimethyl Taurate Copolymer, Isohexadecane | 0.50 |
| Polysorbate 20 | 0.50 |
| Sodium Benzoate | 0.30 |
| Potassium Sorbate | 0.30 |
| Tetrasodium Glutamate Diacetate, Sodium Hydroxide, Water | 0.25 |
| Sodium Hydroxide | 0.14 |
| Rubus Fruticosus Fruit Extract, Propanediol, Water, Citric Acid | 1.50 |
| Water | q.s. to 100 |

## Claims

1. Cosmetic and dermatological use of a *Rubus fruticosus* fruit extract (RFE) for increasing adipocytic mass by topical application.

2. Cosmetic and dermatological use according to claim 1, **characterized in that** the increase of adipocytic mass has a volume increasing effect of the adipose tissue.

3. Cosmetic and dermatological use according to claims 1 or 2, **characterized in that** the topical application is to the breasts, and/or the low neckline, and/or the buttocks, and/or the face, and/or the lips, and/or the cheeks, and/or the eyelids, and/or the hands of a person in need.

4. Cosmetic and dermatological use according to any one of claims 1 to 3, **characterized in that** the RFE is obtained from dried and triturated fruits.

5. Cosmetic and dermatological use according to any one of claims 1 to 4, **characterized in that** the RFE is a solution in the extracting solvent.

6. Cosmetic and dermatological use according to any one of claims 1 to 4, **characterized in that** the RFE is a dried extract.

7. Cosmetic and dermatological use according to any one of claims 1 to 6, **characterized in that** the RFE is obtained by an extraction process comprising the following steps:
a) contacting *Rubus fruticosus* fruits with the extraction solvent forming the extraction mixture,
b) extracting the *Rubus fruticosus* fruits with the extraction solvent at a temperature higher than room temperature, and optionally
c) macerating the extraction mixture at room temperature.

8. Cosmetic and dermatological use according to claim 7, **characterized in that** the extraction solvent is selected from water, alcohols, polyalcohols, oils, and mixtures thereof.

9. Cosmetic and dermatological use according to any one of claims 1 to 8, **characterized in that** the RFE is present in a cosmetic composition.

10. Cosmetic and dermatological use according to claim 9, **characterized in that** the cosmetic composition comprises the RFE and a cosmetically acceptable component.

11. Cosmetic and dermatological use according to claim 10, **characterized in that** the cosmetic composition comprises between 0.001 wt% and 1 wt% of the RFE, as solid, and between 99 wt% and 99.999 wt% of a cosmetically acceptable component, where the percentages of the components are adjusted so that the balance is 100%.

12. Cosmetic and dermatological use according to claim 10 or 11, **characterized in that** the cosmetically acceptable component is selected from a cosmetically acceptable vehicle, a cosmetic ingredient, and mixtures thereof.

13. Cosmetic and dermatological use according to claim 12, **characterized in that** the cosmetic composition comprises:
a) between 0.001 wt% and 1 wt% of RFE, as solid,
b) between 5 wt% and 50 wt% of at least one additional cosmetic ingredient, preferably between 10 wt% and 45 wt%, more preferably between 15 wt% and 35 wt%, and even more preferably between 20 wt% and 25 wt%, and
c) between 49 wt% and 94 wt% of a cosmetically acceptable vehicle, preferably between 50 wt% and 90 wt%, more preferably between 55 wt% and 87 wt%, and even more preferably between 65 wt% and 85 wt%,
where the percentages of the components are adjusted so that the balance is 100%.

14. Cosmetic and dermatological method for increasing adipocytic mass in adipose tissue comprising the step of topically applying to skin an RFE or a cosmetic composition comprising an RFE.

15. Cosmetic and dermatological method according to claim 14, **characterized in that** it comprises the step of topically applying to the bust, and/or the low neckline, and/or the buttocks, and/or the face, and/or the lips, and/or the cheeks, and/or the eyelids, and/or the hands, the RFE or a cosmetic composition comprising the RFE.
